# EUROPEAN PATENT APPLICATION

(11) **EP 4 011 336 A2**
(43) Date of publication of application: **15.06.2022**
(21) Application number: 21208675.5
(22) Date of filing: 17.11.2021
(51) Int. Cl.: A61F 5/14, A43B 17/00, A43B 7/14, A43B 7/16, A43B 7/24

(54) **IMPROVED ORTHOTICS**

(30) Priority: 18.11.2020 AU 2020904253
(71) Applicant: Kinetic Orthotics Pty Ltd, Marcoola, Queensland 4564 (AU)
(72) Inventor: Everson, Dan, Marcoola, Queensland, 4564 (AU)
(74) Representative: Page, White & Farrer Germany LLP

(57) **Abstract**

The present invention relates to an orthotic (10) for a foot, the orthotic (10) having a top side (14) and a bottom side and further comprising a top side raised medial arch structure (16) and a raised lateral arch structure (18) and a flat midfoot section (20), to a half foot orthotic with the same features, to an orthotic for a foot having a top side valgus and/or varus wedge and a first metatarsal indentation, and to related methods.

## Description

### FIELD OF THE INVENTION

The present invention relates to orthotics and related methods, and in particular to an orthotic for a foot having top-side medial and lateral arch structures along with a flat midfoot section, and to an orthotic for a foot having top-side valgus and/or varus wedges and a first metatarsal indentation.

### BACKGROUND OF THE INVENTION

Orthotics for feet are artificial support devices used in the treatment of foot disorders. Such orthotics are often provided as an insert for placement within a patient's shoes. They generally comprise a base with one or more structures disposed thereon, often raised structures on the top and bottom side. Traditionally, orthotic devices are made with structures to provide support with consideration given only to the contour and position of the foot when stationary, for example to redistribute loads imparted on parts of a user's foot whilst standing.

It is only relatively recently that orthotics have begun to be developed with consideration given to the position and contour of the foot when in locomotion. For example, applicant's Australian Patent No. 2012262646 describes a process in which a patient's foot is assessed using functional tests, including with consideration to movement typically experienced during locomotion, and prescribing an orthotic device based thereon and selected from predetermined orthotic designs. More recent orthotic devices are described in applicant's Australian Patent Application No. 2017325102, including orthotic devices defined by a number of structures such as a raised point medial to the calcaneus of a foot (e.g. 'zone 1'), a raised point in the cuboid area of the foot ('zone 2') and a groove posterior of the medial cuneiform curving through the midfoot region to the base of the first metatarsal (e.g. 'zone 3'). The midfoot region in particular is an area that is commonly supported by structures in a variety of different orthotic devices. For example, raised support in the midfoot region is thought to assist natural supination of the foot. Also, a midfoot groove is thought to lessen pressure on the fascial chord of the foot to facilitate a natural windlass mechanism.

Prescribing and using an orthotic device for the treatment of foot disorders often remains a process of trial and error, and at times there is no orthotic device available that may satisfactorily treat a given patient's foot disorder, or offer certain desired benefits to a user.

There exists a need to overcome, or alleviate, one or more of the difficulties or deficiencies associated with the prior art. Thus, the present invention seeks to provide an orthotic device that provides advantages over the prior art, or that may be used to treat any given patient more satisfactorily, or at least to provide a useful alternative.

### SUMMARY OF THE INVENTION

In one aspect, the present invention provides an orthotic for a foot, the orthotic having a top side and a bottom side and further comprising: (a) a raised medial arch structure located on the top side of the orthotic at a position corresponding to the medial longitudinal arch or part thereof of a user's foot; (b) a raised lateral arch structure located on the top side of the orthotic at a position corresponding to the lateral longitudinal arch or part thereof of a user's foot; and (c) a flat midfoot section located on the top side of the orthotic between the medial and lateral arch structures at a position corresponding to the midfoot region or part thereof of a user's foot.

By an "orthotic" for a foot is meant an artificial device for use in improving the operation of a foot by fitment about the sole of a user's foot, which comprises a base which may be shaped to have, or have disposed thereon, one or more structures, generally raised or indented, intended to interact in a specific way, generally supportive, pressure-applying or stimulating, with a particular part of the foot. An orthotic may be by way of a removable insert for footwear or be formed as an integral footwear part, for example formed as a shoe insole, or otherwise. Accordingly, a "top side" of the orthotic is generally that which faces the sole of a user's foot while a "bottom side" of the orthotic is generally that which faces away.

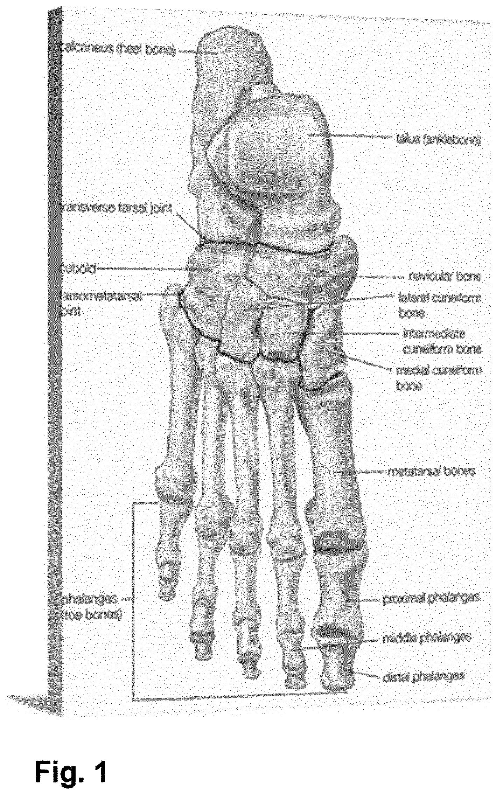

Generally speaking, a person's foot may be thought of as comprising three regions: the forefoot, the midfoot and the hindfoot. These regions may be defined by the bones of the foot. The bones, tendons and muscles of the foot will be known to a person ordinarily skilled in the art. For reference, a pictorial representation of the bones of the foot is provided in Figure 1. The forefoot may include the metatarsals and the phalanges. In that sense, the forefoot region of an orthotic may be considered to be corresponding to the region of the foot extending between the base of the metatarsals and the ends of the distal phalanges, or in other words, extending anterior of the base of the metatarsals or of the tarsometatarsal joint. The midfoot may include the navicular, cuboid and cuneiforms. In that sense, the midfoot region of an orthotic may be considered to be corresponding to the region of the foot extending between posterior of the navicular and cuboid and the base of the metatarsals, or in other words, extending between the transverse tarsal joint and the tarsometatarsal joint. The midfoot region may further be considered to be comprised of two sub-regions: the anterior midfoot subregion and the posterior midfoot subregion. The anterior midfoot subregion may be considered to be corresponding to the region of the foot extending between the naviculo-cuneiform joint (taken to include as extrapolated across the cuboid) and the tarsometatarsal joint. The posterior midfoot subregion of an orthotic may be considered to be corresponding to the region of the foot extending between the transverse tarsal joint and the naviculo-cuneiform joint (again, as extrapolated). Lastly, the hindfoot may include the talus and calcaneus. In that sense, the hindfoot region of an orthotic may be considered to be corresponding to the region of the foot extending between the end of the calcaneus and the navicular and cuboid, or in other words, extending posterior of the transverse tarsal joint.

The shape and function of a person's foot generally defines three arches: the transverse arch, the medial longitudinal arch, and the lateral longitudinal arch. Generally speaking, the transverse arch extends across the foot along a line corresponding to the metatarsal heads. The medial longitudinal arch generally extends along a line of the foot from the first metatarsal head to the centre of the heel, or calcaneus, while the lateral longitudinal arch generally extends along a line of the foot from the fifth metatarsal head, again to the centre of the heel, or calcaneus. Thus, the raised medial and lateral arch structures that are located at a position corresponding to the medial and lateral longitudinal arches or part thereof of a user's foot, respectively, are generally disposed on the orthotic extending along a line corresponding to the first metatarsal head of the foot to the centre of the heel, or calcaneus, or along a line corresponding to the fifth metatarsal head of the foot, again to the centre of the heel, or calcaneus, respectively, or part thereof.

In that sense, the medial and lateral longitudinal arches generally extend through and define the medial and lateral edges of the midfoot region. Thus, by a "flat" midfoot section is generally meant that the base of the orthotic contains no structures intended to interact in a specific support-, pressure- or stimulation-giving way with the corresponding midfoot region of a user's foot, any different to the interaction of the foot with the insole of a normal shoe or other footwear. In other words, there is an absence of structures in the midfoot section; it is structure-less, just like the insole of a normal shoe. In preferred embodiments, the flat midfoot section is located at a position corresponding to the posterior midfoot subregion of a user's foot. In more preferred embodiments, the flat midfoot section is located at a position corresponding to the midfoot region of a user's foot, i.e. both the posterior and anterior midfoot subregions.

The present invention arises from consideration of the operation of the muscular-skeletal and neuromuscular systems of the foot to develop an orthotic that offers support, pressure and/or stimulation, or the absence thereof as the case may be, at various positions of a user's foot, to improve the operation of the foot, particularly during locomotion. The inventor has alighted upon the surprising result that the combination of the flat midfoot section and the medial and lateral arch structures give rise to certain advantages, particularly improvements in the functional position of the foot during locomotion and especially to the gait cycle. Improvement is achieved in particular by providing support along the medial and lateral arches to position and stabilise the foot against excessive protonation or supination during the loading response and mid-stance phases of the gait cycle. This provides alignment, and assists the plantar aponeurosis to maintain the longitudinal arches, for example with a certain pre-tension, in preparedness for an efficient heel rise and terminal stance phase. The arch supports also function to assist the user's weight to roll forward through the foot directed towards the hallux during the heel rise phase for correct weight distribution, and encourages tightening of the plantar fascia around the metatarsal heads during the windlass effect for greater efficiency and improved timing. The flat midfoot section, or absence of midfoot structure, further assists by protecting against pressure or stimulation of the plantar fascia during the windlass effect. This in particular goes against conventional thinking in the art, which holds the general consensus that orthotics often require midfoot structure to assist with natural supination, or to reduce pressure on the plantar fascia. The present inventor has found that midfoot structure is not required, and in many cases may be detrimental, by causing discomfort and interfering with the natural tightening of the plantar fascia thereby reducing the efficiency of the windlass effect, including as otherwise encouraged by the arch supports.

Accordingly, it may be considered that the general purpose of the structures and sections described herein is to provide support, pressure and/or stimulation (or the absence thereof, as the case may be), at various positions of a user's foot, in order to improve the operation of, or optimise the normal operation of, a user's foot, particularly during locomotion.

In preferred embodiments the medial arch structure is located at a position corresponding to anterior of the medial side of the calcaneus of a user's foot and extending to the first metatarsal zone. More preferably, the medial arch structure extends to the first metatarsal base; i.e. is located at a position corresponding to anterior of the medial side of the calcaneus of a user's foot and extending to the first metatarsal base.

Also in preferred embodiments, the lateral arch structure is located at a position corresponding to posterior of the lateral side of the cuboid of a user's foot and extending to the fifth metatarsal head or the fifth metatarsal base.

More preferably, the lateral arch structure comprises first and second substructures, wherein the first substructure is located at a position corresponding to posterior of the lateral side of the cuboid of a user's foot and extending to the fifth metatarsal base, and wherein the second substructure is located at a position corresponding to the fifth metatarsal zone of a user's foot and extending to the fifth metatarsal head.

In more preferred embodiments, the lateral arch structure is located at a position corresponding to posterior of the lateral side of the cuboid of a user's foot and extending to the fifth metatarsal base.

In preferred embodiments, the height of the raised structures is between about 2 mm to about 40 mm, preferably about 10 mm to about 35 mm and more preferably about 15 mm to about 30 mm. Generally speaking, the raised structures will preferably be sloped and, at least insofar as the medial and lateral arch structures are concerned, will slope upwardly towards the medial and lateral perimeters of the orthotic, respectively.

Preferably, the raised structures include a substantially semicircular arc about a respective perimeter of the orthotic and about which the height of the raised structures peak. For example, preferably the medial arch structure includes two raised substantially semicircular arcs, a first located at a position corresponding to anterior of the medial side of the calcaneus of a user's foot and a second located at a position corresponding to the navicular of a user's foot. Given the proximity of the respective positions of the two substantially semicircular arcs, they may be conjoined.

Alternatively, and also preferably, the medial arch structure includes one substantially semicircular arc, a first located at a position corresponding to anterior of the medial side of the calcaneus of a user's foot. Similarly, the lateral arch structure includes a substantially semicircular arc located at a position corresponding to posterior of the lateral side of the cuboid of a user's foot.

When included, the arcs may have a diameter of from 2 mm to 40 mm, for example 2, 5, 10, 15, 20, 25, 30, 35 or 40 mm, or any diameter there between. Preferably, the substantially semicircular arcs about which the height of the raised structures peak may be as hereinbefore described; between about 2 mm to 40 mm.

The orthotic may include a number of other structures so long as the raised medial and lateral arch structures, and the flat midfoot section, are retained. For example, the orthotic may include a heel structure (e.g. a pitched heel), a flexion zone, a first metatarsal indentation and/or a sulci structure as described in applicant's Australian Patent Application No. 2017325102 (i.e. 'zone 7', 'zone 8', 'zone 4', 'zone 6', respectively). The relevant disclosure of applicant's Australian Patent Application No. 2017325102 is hereby incorporated herein by reference.

In one embodiment, the orthotic further comprises a raised metatarsal pad located on the top side of the orthotic at a position corresponding to the metatarsals of a user's foot or part thereof. In one embodiment, the metatarsal pad may extend to the anterior midfoot subregion or part thereof i.e. when the flat midfoot section is located at a position corresponding to the posterior midfoot subregion of a user's foot. In this embodiment, when present, preferably the metatarsal pad is located at a position corresponding to at least the second, third and fourth metatarsal zones, and preferably metatarsal heads, of a user's foot and extends to the head of at least the lateral cuneiform of a user's foot; i.e. is approximately wedge-shaped in the posterior direction. In preferred embodiments when the flat midfoot section is located at a position corresponding to the midfoot region of a user's foot, the metatarsal pad, when present, does not extend to the midfoot region, but rather extends only as far as one or more metatarsal bases.

The orthotic may further comprise a first metatarsal indentation located on the top side of the orthotic at a position corresponding to the first metatarsal head, to receive the medial side ball of the foot. When present, the first metatarsal indentation will preferably be substantially circular or elliptical with a diameter corresponding to the diameter of the medial side ball of the foot, generally between 25 to 50 mm, and will preferably be comparatively shallow, often between about 2 mm to 5 mm in depth.

The orthotic may further comprise a raised heel structure located on the top side of the orthotic at a position corresponding to posterior of the calcaneus of a user's foot. When present, preferably, the heel structure is located at a position corresponding to anterior of the medial side of the calcaneus of a user's foot and extending around through posterior of the calcaneus to posterior of the lateral side of the cuboid of a user's foot. When present, preferably, the heel structure is sloped upwardly in a direction from the centre of the calcaneus of a user's foot towards the perimeter of the orthotic, so as to form a 'cup' in which the heel of a user's foot may fit. In the preferred embodiments of the medial and lateral arch structures as herein described, preferably the heel structure when present meets and integrates with the medial arch structure and the lateral arch structure. The height of the heel structure may be between about 2 mm to about 40 mm, preferably about 5 mm to about 25 mm and more preferably about 10 mm to about 15 mm.

The orthotic may further comprise a raised forefoot structure located on the top side of the orthotic at a position corresponding to the first metatarsal head of a user's foot and extending to the end of the first distal phalange. When present, the height of the raised forefoot structure may be less than the medial and lateral arch structures; preferably between about 0.5 mm to 5 mm, preferably about 1 mm to 2 mm.

The orthotic may further comprise an indented forefoot structure located on the top side of the orthotic at a position corresponding to the fifth metatarsal head of a user's foot and extending to the end of the fifth distal phalange. When present, the indentation may be by an amount of between about 0.5 mm to 5 mm, preferably 1 mm to 3 mm and more preferably about 1 or 2 mm.

In one preferred embodiment, the orthotic further comprises at least a first metatarsal indentation, a raised heel structure and an indented forefoot structure, each located on the top side of the orthotic, as hereinbefore described.

The orthotic may further comprise a valgus wedge and/or a varus wedge, each located on the top side of the orthotic at a position corresponding to the forefoot region or part thereof of a user's foot. A valgus wedge is typically pitched upwardly to the lateral side of the foot, while a varus wedge is typically pitched upwardly to the medial side of the foot. When present, the valgus wedge and varus wedge may be pitched to a height of between about 2 mm to about 15 mm, preferably about 5 mm to about 10 mm, and may be pitched with an angle of about 3 degrees to about 15 degrees, preferably about 5 degrees to about 10 degrees.

In another preferred embodiment, the orthotic further comprises at least a valgus wedge or a varus wedge, and a first metatarsal indentation, each located on the top side of the orthotic in the forefoot region of a user's foot, as hereinbefore described.

A particular benefit is arising from this combination of the valgus wedge and/or a varus wedge and a first metatarsal indentation. The present inventor has found that the combination of these features results in improvements in the functional position of the foot during locomotion and particularly to the gait cycle. Improvement is achieved in particular by the valgus or varus wedge providing support along either the lateral or medial forefoot region, respectively, either or both as may be required for a given patient, in such a way as to direct the user's weight across the optimal load-bearing parts of the foot as the user's weight rolls forwards through the foot during the heel rise and terminal stance phases of gait cycle, to the hallux for toe-off. The first metatarsal indentation further assists by providing an area for the medial side ball of the foot to be received as it bulges, as the plantar fascia tensions around the metatarsal heads. This assists with alignment of the first metatarsal head and hallux for efficient toe-off and prevents the bulging ball of the foot from raising the medial side of the forefoot during the heel rise phase to, in the case of a valgus wedge, work against the valgus wedge, and in the case of a varus wedge, work with the wedge potentially directing the user's weight out of alignment.

Accordingly, in another aspect, the present invention provides an orthotic for a foot, the orthotic having a top side and a bottom side and further comprising: (a) a valgus wedge located on the top side of the orthotic at a position corresponding to the forefoot region or part thereof of a user's foot and pitched upwardly to the lateral side of the foot, and/or a varus wedge located on the top side of the orthotic at a position corresponding to the forefoot region or part thereof of a user's foot and pitched upwardly to the medial side of the foot; and (b) a first metatarsal indentation located on the top side of the orthotic at a position corresponding to the first metatarsal head, to receive the medial side ball of the foot.

The valgus wedge, varus wedge and first metatarsal indentation may be as hereinbefore described. The orthotic may further comprise any one or more of the other structures as hereinbefore described, as applicable, including the raised medial and/or lateral arch supports and/or the flat midfoot section. Preferably, at least the flat midfoot section is included.

The orthotics of the present invention find particular utility in treating foot disorders. Accordingly, in a further aspect, the present invention provides a method for treating a foot disorder, comprising prescribing to a user an orthotic as herein described. Of course, this includes also a method for treating a foot disorder comprising using an orthotic as herein described. This may also include a method for correcting the gait cycle of a user, and in particular the heel rise and terminal stance phases facilitated by the windlass effect.

The orthotic of the present invention finds utility not only for treatment of foot disorders, but also for optimising or improving the normal function of a foot during locomotion; that is, even when there may be no particular prescribable or known treatable disorder. For example, the present invention finds particular utility in relation to the dancing arts, e.g. ballet. The orthotic of the present invention also finds utility in other activities, e.g. sport, and may be used for example by athletes. Accordingly, in a further aspect, the present invention relates to a method for optimising the normal function of a foot during locomotion, comprising using an orthotic as herein described. This may include a method for optimising or improving the gait cycle of a user, and in particular the heel rise and terminal stance phases facilitated by the windlass effect.

For certain applications, for example when used in the dancing arts, and in particular ballet, preferably the orthotic is less than half the length of the foot. Accordingly, in another aspect, the present invention provides an orthotic for a foot, having a top side and a bottom side, the orthotic being less than half the length of the foot and comprising: (a) a raised medial arch structure located on the top side of the orthotic at a position corresponding to the medial longitudinal arch or part thereof of a user's foot; (b) a raised lateral arch structure located on the top side of the orthotic at a position corresponding to the lateral longitudinal arch or part thereof of a user's foot; and (c) a flat midfoot section located on the top side of the orthotic between the medial and lateral arch structures at a position corresponding to the midfoot region or part thereof of a user's foot.

The forefoot region of a foot, which includes elongate metatarsals, is comparatively longer than the midfoot and hindfoot regions - about the same length as the midfoot and hindfoot regions combined. Accordingly, half-foot distance may generally be defined to correspond to the tarsometatarsal joint, or alternatively across a transverse line of the foot taken from the first metatarsal base. In that sense, an orthotic that is less than half the length of the foot may generally be considered to correspond to the region posterior of the half-foot distance.

In this aspect, the medial and lateral structures and flat midfoot section may be as herein described including in their preferred embodiments, as applicable to an orthotic that is less than half the length of the foot.

In this specification, the term 'comprises' and its variants are not intended to exclude the presence of other integers, components or steps.

In this specification, reference to any prior art in the specification is not and should not be taken as an acknowledgement or any form of suggestion that this prior art forms part of the common general knowledge in Australia or any other jurisdiction or that this prior art could reasonably expected to be combined by a person skilled in the art.

The present invention will now be more fully described with reference to the accompanying Examples and Drawings. It should be understood, however, that the description following is illustrative only and should not be taken in any way as a restriction on the generality of the invention described above.

### BRIEF DESCRIPTION OF THE DRAWINGS/FIGURES

- Figure 1: is a pictorial representation of the bones of a foot.
- Figure 2: is a top view of an orthotic of the present invention including medial and lateral arch structures, no midfoot structure, and other features.
- Figure 3: is a top isometric view of an orthotic of the present invention including medial and lateral arch structures, no midfoot structure, and other features.
- Figure 4: is a toe-end view of an orthotic of the present invention including medial and lateral arch structures, no midfoot structure, and other features.

### DETAILED DESCRIPTION OF THE INVENTION

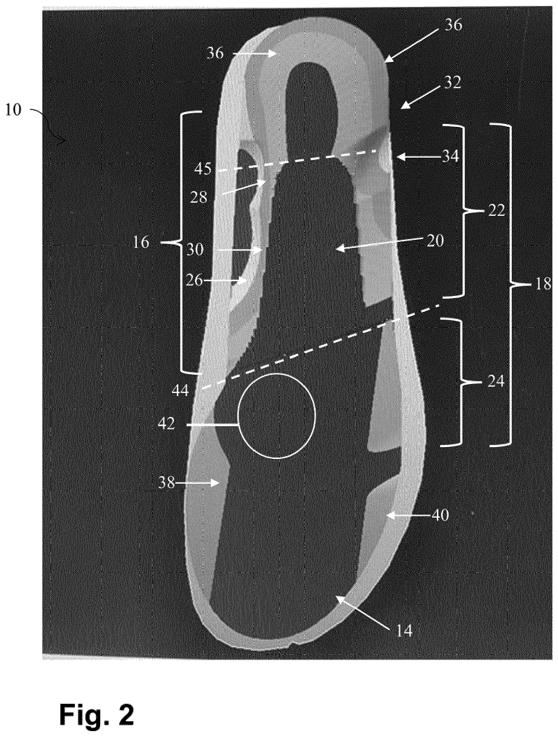

An orthotic of the present invention is shown in Figure 2. The orthotic 10 is generally in the shape of a foot corresponding to a user's foot and e.g. for placement in a user's shoes. The orthotic includes a base with a top side 14 and a bottom side (underneath; not shown).

Disposed on the top side of the orthotic 10 is a raised medial arch structure 16 located at a position corresponding to the medial longitudinal arch or part thereof of a user's foot. As is generally appreciated, the raised medial arch structure is generally disposed on the orthotic extending along a line corresponding to the medial arch of a user's foot, running between the first metatarsal head of the foot to the centre of the heel, or calcaneus. In this embodiment, the medial arch structure 16 is located at a position corresponding to anterior of the medial side of the calcaneus of a user's foot and extending to the first metatarsal base. The medial arch structure is sloped upwardly from the base of the orthotic towards the medial perimeter 26 of the orthotic, and includes semicircular arcs about the perimeter where the raised structure reaches peak height; the first 28 being located at a position corresponding to anterior of the medial side of the calcaneus of a user's foot, and the second 30 being located at a position corresponding to the navicular of a user's foot. Given the proximity, the semicircular arcs are conjoined. The height of the raised medial arch structure 16 at first semicircular arc 28 from base to peak is about 25 mm.

Also disposed on the top side of the orthotic 10 is a raised lateral arch structure 18 located at a position corresponding to the lateral longitudinal arch or part thereof of a user's foot. As is generally appreciated, the raised lateral arch structure is generally disposed on the orthotic extending along a line corresponding to the lateral arch of a user's foot, running between the fifth metatarsal head of the foot to the centre of the heel, or calcaneus. In this embodiment, the lateral arch structure comprises first and second substructures, 22 and 24, respectively. The first substructure 22 is located at a position corresponding to posterior of the lateral side of the cuboid of a user's foot and extending to the fifth metatarsal base. The second substructure 24 is located at a position corresponding to the fifth metatarsal zone of a user's foot and extending to the fifth metatarsal head. In other preferred embodiments, only the first substructure 22 may be included. The lateral arch structure is sloped upwardly from the base 12 towards the lateral perimeter 32 of the orthotic, and includes a semicircular arc 34 about the perimeter where the raised structure reaches peak height, being located at a position corresponding to posterior of the lateral side of the cuboid of a user's foot. The height of the raised lateral arch structure 18 at the a semicircular arc 34 from base to peak is about 15 mm.

The orthotic 10 also includes a flat midfoot section 20 located on the top side between the medial and lateral arch structures 16 and 18, respectively, and in the area between the lines 44 and 45, at a position corresponding to the midfoot region or part thereof of a user's foot. That is, there are no structures disposed on the base of the orthotic in this region.

This embodiment also includes, on the top side of the orthotic 10, a raised heel structure 36 located at a position corresponding to anterior of the medial side of the calcaneus of a user's foot and extending around through posterior of the calcaneus to posterior of the lateral side of the cuboid of a user's foot. The heel structure is sloped upwardly in a direction from the centre of the calcaneus of a user's foot towards the perimeter of the orthotic, so as to form a 'cup' in which the heel of a user's foot may fit. The heel structure meets and integrates with both the medial arch structure 16 and the lateral arch structure 18. The height of the heel structure is uniformly about 10 mm.

This embodiment also includes, on the top side of the orthotic 10, a raised forefoot structure 38 located at a position corresponding to the first metatarsal head of a user's foot and extending to the end of the first distal phalange. The height is about 4 mm. Also included is an indented forefoot structure 40 located at a position corresponding to the fifth metatarsal head of a user's foot and extending to the end of the fifth distal phalange. The height is about 1-2 mm. Also included is a first metatarsal indentation 42 located on the top side of the orthotic at a position corresponding to the first metatarsal head, to receive the medial side ball of the foot. The depth is about 2 mm.

It will be appreciated that a half-foot embodiment is applicable, for example if the orthotic 10 were to be terminated about the line 44 (which is located at a position roughly corresponding with the tarsometatarsal joint of a user's foot).

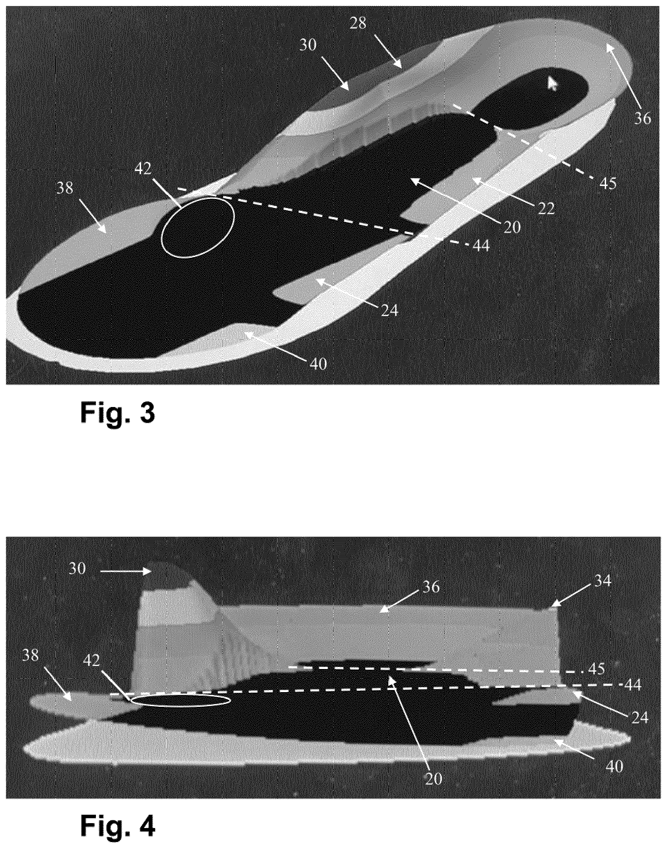

Figure 3 shows the same embodiment of the orthotic 10. From this viewpoint, the height of the raised medial arch structure is clear, indicated by the first and second semicircular arcs 28 (being located at a position corresponding to anterior of the medial side of the calcaneus of a user's foot) and 30 (being located at a position corresponding to the navicular of a user's foot), as well as their sloped and conjoined formation. Similarly, the height of the raised lateral arch structure is visible indicated by the first and second substructures, 22 (located at a position corresponding to posterior of the lateral cuboid of a user's foot and extending to the fifth metatarsal base) and 24 (located at a position corresponding to the fifth metatarsal zone of a user's foot and extending to the fifth metatarsal head). The semicircular arc 34 is also indicated corresponding to posterior of the lateral side of the cuboid of a user's foot. The raised heel structure 36 (located at a position corresponding to anterior of the medial side of the calcaneus of a user's foot and extending around through posterior of the calcaneus to posterior of the lateral side of the cuboid of a user's foot) is also seen including its formation of a 'cup' in which the heel of a user's foot may fit. Also indicated is the flat midfoot section 20 (between the medial and lateral arch structures and the lines 44 and 45) the raised forefoot structure 38 (located at a position corresponding to the first metatarsal head of a user's foot and extending to the end of the first distal phalange), the indented forefoot structure 40 (located at a position corresponding to the fifth metatarsal head of a user's foot and extending to the end of the fifth distal phalange) and the first metatarsal indentation 42 (located on the top side of the orthotic at a position corresponding to the first metatarsal head, to receive the medial side ball of the foot).

Figure 4 shows the same embodiment of the orthotic 10. From this viewpoint, the flat midfoot section 20 located on the top side of the orthotic (between the medial and lateral arch structures and the lines 44 and 45), located at a position corresponding to the midfoot region or part thereof of a user's foot, is clear. That is, it can be seen that there are no structures disposed on the base of the orthotic in this region. Also indicated is the raised heel structure 36, the raised forefoot structure 38, the indented forefoot structure 40, and the first metatarsal indentation 42.

It will be appreciated, especially from the viewpoint of Figure 4, that one or both of a valgus wedge and a varus wedge, each located on the top side of the orthotic at a position corresponding to the forefoot region or part thereof of a user's foot. A representative valgus wedge is envisioned pitched upwardly to the lateral side of the foot, while a varus wedge is envisioned pitched upwardly to the medial side of the foot to a height of about 10 mm and pitched with an angle of about 6 degrees. A varus wedge is particularly preferred, located in about the region of the indented forefoot structure 40, especially when accompanied by a first metatarsal indentation 42.

### Clinical Research

An orthotic 10 as embodied in the figures and described herein has been privately trialled in clinical research involving over 100 patients.

In the results, 98% of patients reported a much greater degree of comfort than their current devices. Most patients also reported little to no discomfort immediately, indicating that the orthotic did not need to be gradually worn-in over an extended period of use as is the suggested guideline of the Australian Podiatry Association. All patients also reported experiencing greater freedom of movement during the gait cycle, and this was accompanied by postural improvement. Patients in the trial group who suffered from functionally-related lower back pain all reported experiencing a greater than 80 percent reduction in their symptoms as assessed on a 1 to 10 perception scale. Similarly, patients in the trial group who suffered from hip and knee pain reported about an 85% reduction in symptoms on a 1 to 10 perception scale. Greater than 95 percent of patients also reported a reduction in foot and heel pain.

The results of the clinical research highlight the benefits of an orthotic having raised medial and lateral arch structures located on the top side of the orthotic and in particular a flat midfoot section there between. It has become apparent that, for many patients, structure in the midfoot region, and particularly in the posterior midfoot region, can cause disruption to the windlass effect and block force transfer through the foot during locomotion which gives rise to discomfort, pain and poor posture. The orthotic of the present invention avoids this disruption to the windlass effect and force transfer blocking, allowing optimal force transfer and functional efficiency during the gait cycle.

Finally, it is to be understood that various alterations, modifications and/or additions may be made without departing from the spirit of the present invention as outlined herein.

## Claims

1. An orthotic for a foot, the orthotic having a top side and a bottom side and further comprising:
(a) a raised medial arch structure located on the top side of the orthotic at a position corresponding to the medial longitudinal arch or part thereof of a user's foot;
(b) a raised lateral arch structure located on the top side of the orthotic at a position corresponding to the lateral longitudinal arch or part thereof of a user's foot; and
(c) a flat midfoot section located on the top side of the orthotic between the medial and lateral arch structures at a position corresponding to the midfoot region or part thereof of a user's foot.

2. The orthotic according to claim 1, wherein the medial arch structure is located at a position corresponding to anterior of the medial side of the calcaneus of a user's foot and extending to the first metatarsal zone, the medial arch structure preferably extending to the first metatarsal base.

3. The orthotic according to claim 1 or 2, wherein the lateral arch structure is located at a position corresponding to posterior of the lateral side of the cuboid of a user's foot and extending to the fifth metatarsal head or the fifth metatarsal base, wherein the lateral arch structure preferably comprises first and second substructures, the first substructure being located at a position corresponding to posterior of the lateral side of the cuboid of a user's foot and extending to the fifth metatarsal base, and the second substructure being located at a position corresponding to the fifth metatarsal zone of a user's foot and extending to the fifth metatarsal head.

4. The orthotic according to any of preceding claims, wherein the raised structures include a substantially semicircular arc about which the height of the raised structures peaks.

5. The orthotic according to claim 4, wherein the medial arch structure includes two raised substantially semicircular arcs, a first located at a position corresponding to anterior of the medial side of the calcaneus of a user's foot and a second located at a position corresponding to the navicular of a user's foot.

6. The orthotic according to claim 4 or 5, wherein the lateral arch structure includes a substantially semicircular arc located at a position corresponding to posterior of the lateral side of the cuboid of a user's foot.

7. The orthotic according to any of preceding claims, further comprising a raised heel structure located on the top side of the orthotic at a position corresponding to posterior of the calcaneus of a user's foot, optionally wherein the heel structure is located at a position corresponding to anterior of the medial side of the calcaneus of a user's foot and extending around through posterior of the calcaneus to posterior of the lateral side of the cuboid of a user's foot, and/or the heel structure is sloped upwardly in a direction from a position corresponding to the centre of the calcaneus of a user's foot towards the perimeter of the orthotic, so as to form a 'cup' in which the heel of a user's foot may fit, further optionally wherein the heel structure meets and integrates with the medial arch structure and the lateral arch structure.

8. The orthotic according to any of preceding claims, further comprising a valgus wedge located on the top side of the orthotic at a position corresponding to the forefoot region or part thereof of a user's foot and pitched upwardly to the lateral side of the foot, and/or a varus wedge located on the top side of the orthotic at a position corresponding to the forefoot region or part thereof of a user's foot and pitched upwardly to the medial side of the foot.

9. The orthotic according to any of preceding claims, further comprising a first metatarsal indentation located on the top side of the orthotic at a position corresponding to the first metatarsal head of a user's foot, to receive the medial side ball of the foot.

10. The orthotic according to any of preceding claims, further comprising a raised forefoot structure located on the top side of the orthotic at a position corresponding to the first metatarsal head of a user's foot and extending to the end of the first distal phalange.

11. The orthotic according to any of preceding claims, further comprising an indented forefoot structure located on the top side of the orthotic at a position corresponding to the fifth metatarsal head of a user's foot and extending to the end of the fifth distal phalange.

12. The orthotic according to any of preceding claims, wherein the midfoot section is located at a position corresponding to the midfoot region of a user's foot.

13. The orthotic according to any of preceding claims, wherein the flat midfoot section is located at a position corresponding to the posterior midfoot subregion of a user's foot, wherein the orthotic preferably further comprises a raised metatarsal pad located on the top side of the orthotic at a position corresponding to at least the second, third and fourth metatarsal zones of a user's foot and extending in a wedge shape to the head of at least the lateral cuneiform of a user's foot.

14. An orthotic for a foot, having a top side and a bottom side, the orthotic being less than half the length of the foot and further comprising:
(a) a raised medial arch structure located on the top side of the orthotic at a position corresponding to the medial longitudinal arch or part thereof of a user's foot;
(b) a raised lateral arch structure located on the top side of the orthotic at a position corresponding to the lateral longitudinal arch or part thereof of a user's foot; and
(c) a flat midfoot section located on the top side of the orthotic between the medial and lateral arch structures at a position corresponding to the midfoot region or part thereof of a user's foot.

15. An orthotic for a foot, the orthotic having a top side and a bottom side and further comprising:
(a) a valgus wedge located on the top side of the orthotic at a position corresponding to the forefoot region or part thereof of a user's foot and pitched upwardly to the lateral side of the foot, and/or a varus wedge located on the top side of the orthotic at a position corresponding to the forefoot region or part thereof of a user's foot and pitched upwardly to the medial side of the foot; and
(b) a first metatarsal indentation located on the top side of the orthotic at a position corresponding to the first metatarsal head, to receive the medial side ball of the foot;
optionally the orthotic further comprising:
(c) a flat midfoot section located on the top side of the orthotic at a position corresponding to the midfoot region or part thereof of a user's foot.

16. A method for treating a foot disorder, comprising prescribing to a user an orthotic according to any of preceding claims, and/or for improving or optimising the normal function of a foot during locomotion, comprising using an orthotic according to any of preceding claims, and/or for correcting the gait cycle or improving or optimising the normal gait cycle, comprising using an orthotic according to any of preceding claims.
